# EUROPÄISCHE PATENTSCHRIFT

(11) **EP 0 706 529 B1**
(45) Veröffentlichungstag und Bekanntmachung des Hinweises auf die Patenterteilung: **21.10.1998**
(21) Anmeldenummer: 94922861.3
(22) Anmeldetag: 22.06.1994
(51) Int. Cl.: C07H 15/04

(54) **VERFAHREN ZUR HERSTELLUNG VON ALKYL- UND/ODER ALKENYLOLIGOGLUCOSIDEN**
METHOD OF PREPARING ALKYL AND/OR ALKENYL OLIGOGLUCOSIDES
PROCEDE DE PREPARATION D'ALKYLOLIGOGLUCOSIDES ET/OU D'ALCENYLOLIGOGLUCOSIDES

(30) Priorität: 01.07.1993 DE 4321840
(43) Veröffentlichungstag der Anmeldung: 17.04.1996
(73) Patentinhaber: Henkel Kommanditgesellschaft auf Aktien, 40191 Düsseldorf (DE)
(72) Erfinder: SCHULZ, Paul, D-42115 Wuppertal (DE); ESKUCHEN, Rainer, D-40764 Langenfeld (DE)
(86) Internationale Anmeldenummer: EP9402036
(87) Internationale Veröffentlichungsnummer: WO9501360

(56) Entgegenhaltungen:
- EP-A- 0 096 917
- EP-A- 0 387 912

## Beschreibung

Die Erfindung betrifft die Verwendung von Glucose einer ausgewählten Korngröße für die Herstellung von Alkyl- und/oder Alkenyloligoglucosiden.

### Stand der Technik

Alkyloligoglykoside, insbesondere Alkyloligoglucoside stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in Skin Care Forum, 1, (Okt.1992), D.Balzer und N.Ripke in Seifen-Öle-Fette-Wachse 118, 894 (1992) und B.Brancq in Seifen-Öle-Fette-Wachse 118, 905 (1992) genannt werden sollen.

Zu ihrer Herstellung geht man üblicherweise von Glucose aus, die in Gegenwart saurer Katalysatoren mit Fettalkoholen acetalisiert wird. Anschließend wird der Katalysator neutralisiert, überschüssiger Fettalkohol abgetrennt und das Produkt gebleicht.

Als Katalysatoren für die Acetalisierung kommen grundsätzlich Säuren in Betracht. Schwefelsäure beispielsweise ist in der Kondensation äußerst wirksam, führt jedoch stets zu sehr dunkelgefärbten Produkten, die sich nicht oder nur schwer aufhellen lassen; zudem wird ein hoher Gehalt an unerwünschten Nebenprodukten, insbesondere Polyglucose, beobachtet.

In der Vergangenheit hat es nicht an Vorschlägen für geeignete saure Katalysatoren gefehlt, mit deren Hilfe man die Produktverteilung und insbesondere die Reaktionsgeschwindigkeit zu steuern hoffte. So ist beispielweise aus den beiden Schriften EP-B1 0 132 043 und EP-B1 0 132 046 (Procter & Gamble) bekannt, daß sich p-Toluolsulfonsäure und insbesondere auch anionische Tenside in saurer Form, wie etwa langkettige Alkylbenzolsulfonsäuren, Alkylsulfonsäuren und Schwefelsäurehalbester von Fettalkoholen und deren Polyglycolethern für diesen Zweck eignen. In ähnlicher Weise beschreibt die US 5,003,057 (Henkel Corp.) die Verwendung von Naphthalinsulfonsäure. Aus der EP-A 0 415 192 ist schließlich die Acetalisierung von Zuckern in Gegenwart von Sulfobernsteinsäure bekannt.

Auch die EP 0096917 beschreibt ein Verfahren zur Herstellung von Alkylglycosiden durch Säurekatalysierte umsetzung von Alkoholen mit Sacchariden, dabei werden bevorzugt feingemahlene monosaccharide mit einer Teilchengröße unterhalb von 300 nm eingeseht.

Alle diese bekannten Verfahren haben jedoch den Nachteil gemeinsam, daß die Reaktionsgeschwindigkeit gemessen an anderen technischen Verfahren nach wie vor unbefriedigend ist und lange Kesselbelegungszeiten resultieren, die die Wirtschaftlichkeit des Verfahrens belasten. Des weiteren geht mit der Länge der Reaktionszeit erfahrungsgemäß auch eine Verschlechterung der Farbqualität einher, so daß auch aus diesem Grunde eine Verkürzung der Reaktionszeit erwünscht ist. Im Hinblick auf die Produktverteilung hat sich die Auswahl der Katalysatorsäure zudem als wenig relevant erwiesen.

Die Aufgabe der Erfindung hat somit darin bestanden, ein verbessertes Verfahren zur Herstellung von Alkyl- und/oder Alkenyloligoglucosiden zu entwickeln, das frei von den geschilderten Nachteilen ist.

### Beschreibung der Erfindung

Gegenstand der Erfindung ist die Verwendung von Glucose mit
a) einer Korngrößenverteilung von mindestens 90 % im Bereich von 20 bis 200 µm sowie
b) einem Feinstkornanteil (< 20 µm) und einem Grobkornanteil (> 500 µm) von jeweils weniger als 10 Gew.-%
für die Herstellung von Alkyl- und/oder Alkenyloligoglykosiden.

Überraschenderweise wurde gefunden, daß die Korngröße der Glucose einen erheblichen Einfluß auf die Geschwindigkeit der Acetalisierung und damit die Farbqualität hat. Gleichzeitig wird im Sinne des erfindungsgemäßen Verfahrens die Selektivität der Acetalisierung in Richtung auf einen erhöhten Anteil an Monoglucosiden und einen verminderten Anteil an höheren Oligomeren signifikant verbessert.

Die Erfindung schließt die Erkenntnis ein, daß Glucose einer breiten Korngrößenverteilung innerhalb des Bereiches von 20 bis 200 µm im Hinblick auf übliche Verdampfungskapazitäten eine optimale Reaktionsgeschwindigkeit zeigt.

Des weiteren hat es sich als vorteilhaft erwiesen, Glucose einer Qualität zu verwenden, die sowohl einen Feinstkornanteil (< 20 µm), als auch einen Grobkornanteil (> 500 µm) von jeweils weniger als 10, vorzugsweise weniger als 5 Gew.-% aufweist. Diese Maßnahme ist insbesondere im Hinblick auf den Einsatz längerkettigerer Fettalkohole vorteilhaft.

### Herstellung der Alkyl- und/oder Alkenyloligoglucoside

Die Herstellung der Alkyl- und/oder Alkenyloligoglucoside durch sauer katalysierte Acetalisierung von Glucose mit Fettalkoholen - gegebenenfalls über die Zwischenstufe der Butylglucoside - ist aus einer Vielzahl von Publikationen bekannt. Stellvertretend sei in diesem Zusammenhang auf die Schriften EP-A1-0 301 298 und WO 90/3977 verwiesen.

Die erfindungsgemäß einzusetzende Glucose stellt ein kommerziell erhältliches Produkt dar, welches jedoch auch aus konventionellen Typen durch einfache Siebklassierung zugänglich ist.

Als Fettalkohole kommen solche mit 6 bis 22, vorzugsweise 8 bis 16 Kohlenstoffatomen in Betracht. Vorzugsweise werden technische C₁₂₋₁₆- oder C₈₋₁₀-Fettalkohole auf Basis Kokos- oder Palmkernöl eingesetzt.

Als saure Katalysatoren kommen tensidische und nichttensidische Systeme in Betracht, beispielsweise Dodecanbenzolsulfonsäure, Naphthalinsulfonsäure, Sulfobernsteinsäure, Sulfoessigsäure, p-Toluolsulfonsäure, Methansulfonsäure und Sulfotriacetin. Die Katalysatoren können in Mengen von 1 bis 10 mEq - bezogen auf die Glucose - eingesetzt werden.

Im Anschluß an die Acetalisierung können die sauren Katalysatoren in bekannter Weise, insbesondere durch Zusatz von Magnesiumoxid und/oder Natriumhydroxidlösung neutralisiert werden. Die Abtrennung des überschüssigen Fettalkohols erfolgt zweckmäßigerweise zweistufig im Sinne einer Grobabreicherung in einem Fallfilm- und einer Feinabreicherung in einem Dünnschichtverdampfer. Anschließend können die resultierenden Alkyl- und/oder Alkenyloligoglykoside mit Wasser angepastet und/oder mit Wasserstoffperoxid gebleicht werden.

### Gewerbliche Anwendbarkeit

Die erfindungsgemäß einzusetzende Glucose zeichnet sich in der Acetalisierung durch eine besonders hohe Reaktionsgeschwindigkeit aus und führt zu vergleichsweise hellfarbigen Produkten mit einem hohen Gehalt an Monoglucosiden.

Die folgenden Beispiele sollen den Gegenstand der Erfindung näher erläutern, ohne ihn darauf einzuschränken.

### Beispiele

a) **Reaktionsgeschwindigkeit**. In einem 1-1-Dreihalskolben mit Rührer, Tropftrichter und Destillationsaufsatz wurden 234 g (1,3 mol) wasserfreie Glucose einer Korngrößenverteilung von 90 % im Bereich von 20 bis 200 bzw. 200 bis 800 µm vorgelegt und mit 1400 g (6,5 mol) C_{12/14} Kokosfettalkohol (Lorol^{(R)} Spezial, Hydroxyzahl: 290; Fa. Henkel KGaA, Düsseldorf/ FRG) versetzt. Die Reaktionsmischung wurde auf 90°C vorgeheizt, ein Vakuum von 20 mbar angelegt und dann über den Tropftrichter innerhalb von 5 min 4 mEq - bezogen auf Glucose - Methansulfonsäure zudosiert. Nach Beendigung der Zugabe (t=0) wurde die Reaktionsmischung auf 110°C erhitzt und die Wasserabscheidung - als Maß für die Reaktionsgeschwindigkeit - in Abhängigkeit der Reaktionszeit verfolgt. Die Ergebnisse sind in Tab.1 zusammengefaßt.
b) **Produktzusammensetzung**. Das rohe Reaktionsprodukt wurde neutralisiert, in eine Vakuumdestillationsapparatur überführt und der überschüssige Fettalkohol bei einer Temperatur von 180°C und einem verminderten Druck von 5 mbar abdestilliert. Anschließend wurden die Produkte mit 1 Gew.-% Wasserstoffperoxid - bezogen auf die Produkte - bei pH - 9 gebleicht. Die Zusammensetzung der Produkte kann Tab.2 entnommen werden.

**Tab.1:**

| Reaktionsgeschwindigkeit | | | | |
|---|---|---|---|---|
| Bsp. | Korngröße µm | t(30) min | t(50) min | t(70) min |
| 1 | 20-200 | 42 | 63 | 100 |
| V1 | 200-800 | 80 | 125 | 160 |
| Legende: t(x) = Zeit bis zur Abscheidung von x % der theoretischen Menge an Reaktionswasser | | | | |

**Tab.2:**

| Produktzusammensetzung Prozentangaben als Gew.-% | | | | | | | |
|---|---|---|---|---|---|---|---|
| Bsp. | Korngröße µm | Monoglucosid (%) | | | | DG % | Rest % |
| | | MG | C12-α-GP | C12-β-GP | C12-GF | | |
| 2 | 20-200 | 59 | 33 | 11 | 2 | 14 | 40 |
| V4 | 200-800 | 51 | 30 | 10 | 1 | 15 | 44 |
| Legende: MG - Monoglucosid DG - Diglucosid C12-α-GP = C12-α-Glucopyranosid C12-β-GP - C12-β-Glucopyranosid C12-GF = C12-Glucofuranosid | | | | | | | |

## Patentansprüche

1. Verwendung von Glucose mit
a) einer Korngrößenverteilung von mindestens 90 % im Bereich von 20 bis 200 µm sowie
b) einem Feinstkornanteil (< 20 µm) und einem Grobkornanteil (> 500 µm) von jeweils weniger als 10 Gew.-%
für die Herstellung von Alkyl- und/oder Alkenyloligoglykosiden.

## Claims

1. The use of glucose with
a) a particle size distribution of at least 90% in the 20 to 200 µm range and
b) a fine-particle component (<20 µm) of less than 10% by weight and a coarse-particle component (>500 µm) of less than 10% by weight
for the production of alkyl and/or alkenyl oligoglycosides.

## Revendications

1. Utilisation de glucose ayant :
a) une répartition granulométrique d'au moins 90 % entre 20 et 200 µm, ainsi que
b) une proportion de grains fins (inférieurs à 20 µm) et une proportion de gros grains(supérieurs à 500 µm) qui sont l'une et l'autres inférieures à 10 % en poids,
pour la fabrication d'alkyl- et/ou alcényloligoglycosides.
